(19) 

Europäisches
Patentamt

European
Patent Office

Office européen
des brevets

(11) **EP 3 936 498 A1**

(12) **EUROPEAN PATENT APPLICATION**
published in accordance with Art. 153(4) EPC

(43) Date of publication:
**12.01.2022 Bulletin 2022/02**

(21) Application number: **20770718.3**

(22) Date of filing: **02.03.2020**

(51) Int Cl.:
*C07C 51/00* (2006.01)      *C07C 53/02* (2006.01)
*C07B 61/00* (2006.01)      *B01J 31/22* (2006.01)
*B01J 31/24* (2006.01)

(86) International application number:
**PCT/JP2020/008680**

(87) International publication number:
**WO 2020/184254 (17.09.2020 Gazette 2020/38)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **08.03.2019 JP 2019042918**
**17.12.2019 JP 2019227277**

(71) Applicant: **NITTO DENKO CORPORATION**
**Ibaraki-shi**
**Osaka 567-8680 (JP)**

(72) Inventors:
 • **MATSUDA Hirokazu**
  **Ibaraki-shi, Osaka 567-8680 (JP)**
 • **KOTAKE Shinya**
  **Ibaraki-shi, Osaka 567-8680 (JP)**

(74) Representative: **Hoffmann Eitle**
**Patent- und Rechtsanwälte PartmbB**
**Arabellastraße 30**
**81925 München (DE)**

(54) **METHOD FOR PRODUCING FORMIC ACID**

(57)    The present invention relates to a method for producing a formic acid including, a first step of allowing carbon dioxide and hydrogen to react with each other in a solution containing a solvent and a catalyst dissolved in the solvent and in the presence of an amine insoluble in the solvent, and allowing a generated formic acid to adsorb to the amine, in which the catalyst contains at least one metal element selected from the group consisting of metal elements belonging to Groups 8, 9, and 10 of a periodic table and the amine is an amine immobilized on a solid.

EP 3 936 498 A1

**Description**

TECHNICAL FIELD

The present invention relates to a method for producing a formic acid.

BACKGROUND ART

Due to problems such as global warming and depletion of fossil fuels, hydrogen energy has been highly expected as next-generation energy.

A formic acid is considered to be an excellent compound as a hydrogen storage material and is attracting attention, because the formic acid requires low energy for dehydrogenation reaction and can be handled easily.

In order to use a formic acid as a hydrogen storage material, it is necessary to obtain a formic acid solution having a high concentration, for reducing a transportation cost. It is necessary to separate and recover a formic acid from a formic acid solution with high efficiency.

Therefore, a method for producing a formic acid from carbon dioxide ($CO_2$) and hydrogen ($H_2$) in the presence of a catalyst has been studied. For example, Patent Literature 1 describes a method for producing a formic acid by a reaction between carbon dioxide and hydrogen in a hydrogenation reactor in the presence of a catalyst containing an element belonging to Groups 8, 9, or 10 of a periodic table and containing a tertiary amine. Non-Patent Literature 1 describes a method for producing a formic acid from carbon dioxide and hydrogen in the presence of a catalyst using an amine supported on a solid.

CITATION LIST

PATENT LITERATURE

Patent Literature 1: specification of US Patent No. 8791297

NON-PATENT LITERATURE

Non-Patent Literature 1: RSC Adv., 2014, 4, 49995-50002

SUMMARY OF INVENTION

TECHNICAL PROBLEM

The technique described in Patent Literature 1 has problems that a complicated operation is required for separation and extraction of a formic acid from a catalyst and a solvent and a large amount of energy is required to recover the formic acid from the obtained formic acid aqueous solution as a two-phase reaction solution is used for extracting a formic acid generated in a liquid amine phase into an aqueous phase.

In the technique described in Non-Patent Literature 1, an amount of formic acid generated per reaction time is small, and concentration of a formic acid solution has not been studied.

Therefore, the present invention provides a method for producing a formic acid in which the formic acid solution can be concentrated with high efficiency by a simple method and the formic acid can be recovered at a high yield.

SOLUTION TO PROBLEM

The present inventors have conducted intensive studies for the purpose of discovering a production method in which a formic acid solution can be concentrated with high efficiency by a simple method and a formic acid can be recovered at a high yield, and as a result, the present invention has been completed.

Means for solving the above problems are as follows.

[1] A method for producing a formic acid, comprising:

a first step of allowing carbon dioxide and hydrogen to react with each other in a solution containing a solvent and a catalyst dissolved in the solvent and in the presence of an amine insoluble in the solvent to generate a formic acid, and allowing the generated formic acid to adsorb to the amine, wherein
the catalyst contains at least one metal element selected from the group consisting of metal elements belonging to Groups 8, 9, and 10 of a periodic table, and
the amine is an amine immobilized on a solid.

[2] The method for producing a formic acid according to [1], further comprising: a second step of separating the amine to which the formic acid is adsorbed and the catalyst-containing solution.
[3] The method for producing a formic acid according to [2], wherein the catalyst-containing solution separated in the second step is reused in the first step.
[4] The method for producing a formic acid according to [2] or [3], further comprising: a third step of heating the amine to which the formic acid is adsorbed to recover the formic acid.
[5] The method for producing a formic acid according to any one of [1] to [4], wherein the metal element is Ru, Ir, Fe, or Co.
[6] The method for producing a formic acid according to any one of [1] to [5], wherein the metal element is Ir or Ru.
[7] The method for producing a formic acid according to any one of [1] to [6], wherein the amine immobilized on the solid is an amine immobilized on a polymer.
[8] The method for producing a formic acid according to [7], wherein the polymer on which the amine is immobilized is polystyrene.

ADVANTAGEOUS EFFECTS OF INVENTION

The present invention allows for providing a method for producing a formic acid in which a formic acid solution can be concentrated with high efficiency by a simple method and a formic acid can be recovered at a high yield.

DESCRIPTION OF EMBODIMENTS

Hereinafter, an embodiment of the present invention will be described in detail.
A method for producing a formic acid according to an embodiment of the present invention includes a first step of allowing carbon dioxide and hydrogen to react with each other in a solution containing a solvent and a catalyst dissolved in the solvent and in the presence of an amine insoluble in the solvent to generate a formic acid, and allowing the generated formic acid to adsorb to the amine, in which the catalyst contains at least one metal element selected from the group consisting of metal elements belonging to Groups 8, 9, and 10 of a periodic table and the amine is an amine immobilized on a solid.
The method for producing a formic acid according to the embodiment of the present invention preferably further includes a second step of separating the amine to which the formic acid is adsorbed and the catalyst-containing solution.
The method for producing a formic acid according to the embodiment of the present invention preferably further includes a third step of heating the amine to which the formic acid is adsorbed to recover the formic acid.

[First Step]

The first step is a step of allowing carbon dioxide and hydrogen to react with each other in a solution containing a solvent and a catalyst dissolved in the solvent and in the presence of an amine insoluble in the solvent to generate a formic acid, and allowing the generated formic acid to adsorb to the amine. In this step, the formic acid generated in the reaction solution is adsorbed to the amine insoluble in the solvent. The catalyst is dissolved in the solvent and the amine is immobilized on a solid, and thus the amine to which the formic acid is adsorbed and the solution containing the catalyst can be separated by a simple method.

(Catalyst)

The catalyst used in the embodiment of the present invention contains at least one metal element selected from the group consisting of metal elements belonging to Groups 8, 9, and 10 of the periodic table (hereinafter, simply referred to as a metal element). Specific examples of the metal element include Fe, Ru, Os, Hs, Co, Ir, Mt, Ni, Pd, Pt, and Ds. Ru, Ir, Fe and Co are preferable, and Ru and Ir are more preferable, from the viewpoint of catalytic performance.
The catalyst used in the embodiment of the present invention is preferably dissolved in water, an organic solvent, or the like, and is more preferably a compound containing a metal element (a metal element compound), since the first step needs to be performed in a solution containing a catalyst dissolved in a solvent.
Examples of the metal element compound include a salt of a metal element with an inorganic acid such as a hydride salt, an oxide salt, a halide salt (such as a chloride salt), a hydroxide salt, a carbonate salt, a hydrogen carbonate salt, a sulfate salt, a nitrate salt, a phosphate salt, a borate salt, a salt of halogen acids, a salt of perhalogen acids, a salt of halous acids, a salt of hypohalous acids, and a thiocyanate salt; a salt of a metal element with an organic acid such as an alkoxide salt, a carboxylate salt (such as an acetate salt and a (meth)acrylate salt), and a sulfonate salt (such as a trifluoromethanesulfonate salt); a salt of a metal element with an organic base such as an amide salt, a sulfonamide salt, and a sulfonimide salt (such as a bis(trifluoromethanesulfonyl)imide salt); a complex salt such as an acetylacetone salt, a hexafluoroacetylacetone salt, a porphyrin salt, a phthalocyanine salt, and a cyclopentadiene salt; complexes or salts containing one or more of a nitrogen compound containing a chain amine, a cyclic amine, an aromatic amine, and the like, a phosphorus compound, a compound containing phosphorus and nitrogen, a sulfur compound, carbon monoxide, carbon dioxide, water, and the like. These compounds may be either a hydrate or an anhydride, and are not limited. Among these, a halide salt, a complex containing a phosphorus compound, a complex containing a nitrogen compound, and a complex or salt containing a compound containing phosphorus and nitrogen are preferable from the viewpoint of further enhancing the efficiency of producing a formic acid.

These may be used alone or in combination of two or more.

For the metal element compound, a commercially available metal element compound can be used, or a metal element compound produced by a known method or the like can also be used. As the known method, for example, a method described in JP-A-2008-184398, and a method described in Angew. Chem. Int. Ed. 2010, 49, 1468-1471 can be used.

An amount of the catalyst to be used is not limited as long as the formic acid can be produced. When the metal element compound is used as the catalyst, an amount of the metal element compound to be used is preferably 0.1 $\mu$mol or more, more preferably 0.5 $\mu$mol or more, and still more preferably 1 $\mu$mol or more with respect to 1 L of the solvent in order to express the sufficient catalytic function. From the viewpoint of cost, the amount is preferably 1 mol or less, more preferably 10 mmol or less, and still more preferably 1 mmol or less. When two or more kinds of the metal element compounds are used, a total amount of the metal element compounds to be used may be within the above range.

(Solvent)

The solvent according to the embodiment of the present invention is not limited as long as the solvent dissolves the catalyst and becomes uniform, and examples of the solvent include water, methanol, ethanol, N,N-dimethylformamide (DMF), dimethyl sulfoxide, tetrahydrofuran, benzene, toluene, and a mixed solvent of these. N,N-dimethylformamide, methanol, tetrahydrofuran, or water is preferably contained, and N,N-dimethylformamide or water is more preferable.

(Amine)

In the method for producing a formic acid according to the embodiment of the present invention, an amine immobilized on a solid is used. The amine needs to be an amine insoluble in a solvent in a solution containing the solvent and the catalyst dissolved in the solvent. That is, the amine needs to be insoluble in the solution in which the catalyst used in the first step is dissolved.

Here, the term "immobilized" refers to a state in which an amine is bound to a solid to be insoluble or immobilized. The term "immobilized" also includes "supported", a state in which an amine is attached to a solid.

The solid is not limited as long as the solid is insoluble in the solution in which the catalyst used in the first step is dissolved, preferably insoluble in water, an organic solvent, or the like, and the amine can be immobilized on a surface of the solid. The solid is preferably one in which the immobilized amine can bond to the surface of the solid directly or via a spacer group, and more preferably one having a functional group capable of chemically bonding to the amine.

Specific examples of the solid include porous particles, polymers, and metal oxides, and a polymer is preferable from the viewpoint that many amine functional groups can be set.

Examples of the porous particles include activated carbon and silica gel.

Examples of the polymer include polystyrene, polyethylene, polypropylene, a polyurethane resin, an acrylic resin, an epoxy resin, and a silicone resin. Polystyrene, polyethylene and the like, which facilitate the introduction of amine functional groups, are preferable, and polystyrene is more preferable.

Examples of the metal oxide include alumina.

A type and a structure of the amine are not limited. In more detail, inorganic amines and organic amines (aliphatic amines, aromatic amines, heterocyclic amines, amidine, guanidine, and the like) may be included.

Specific examples of the amine immobilized on a solid include compounds in which amines such as ammonia, dimethylamine, diethylamine, diisopropylamine, trimethylamine, triethylamine, N,N-dimethylaniline, N,N-diisopropylethylamine (DIEA), 1,4-diazabicyclo[2.2.2]octane (DABCO), 1-methylimidazole, pyridine, 4-dimethylaminopyridine (DMAP), 2,6-lutidine, 2,4,6-collidine, 1,5-diazabicyclo[4.3.0]-5-nonene (DBN), 1,8-diazabicyclo[5.4.0]-7-undecene (DBU), alkanol-8-diazabicyclo[5.4.0]-7-undecene (DBUOH), 1,5,7-triazabicyclo[4.4.0]deca-5-ene (TBD), and 7-methyl-1,5,7-triazabicyclo[4.4.0]deca-5-ene (MTBD) are immobilized on a solid. Among these, compounds in which DMAP, DBU, DBN, TBD, or MTBD are immobilized on a solid are preferable from the viewpoint of further increasing the yield of a formic acid, and DMAP (PS-DMAP) immobilized on polystyrene or DBU (PS-DBU) immobilized on polystyrene is more preferable, as a formic acid and a catalyst can be separated only by filtration and can be easily recovered at the time of distillation.

As the amine, a commercially available amine may be used, or an amine produced by a known method or the like may be used. These may be used alone or in combination of two or more.

An amount of the amine to be used is not limited, but is preferably 5 mmol or more, more preferably 25 mmol or more, and still more preferably 50 mmol or more in terms of nitrogen atom weight with respect to 1 L of the solvent from the viewpoint of efficiently recovering the generated formic acid. From the viewpoint of cost, the amount of the amine to be used is preferably 50 mol or less, more preferably 25 mol or less, and still more preferably 5 mol or less in terms of nitrogen atom weight with respect to 1 L of the solvent.

Here, the nitrogen atomic weight means the amount of nitrogen atoms contained in the amine. For example, in the case of DMAP or DBU, two nitrogen atoms are contained in one amine functional group.

When two or more kinds of amines are used, the total amount of the amines to be used may be within the above range.

(Carbon Dioxide and Hydrogen)

As the hydrogen used in the embodiment of the present invention, both of a hydrogen gas cylinder and liquid hydrogen can be used. As a hydrogen supply source, for example, hydrogen generated in a smelting process of iron making, hydrogen generated in a production process of Soda, or the like can be used. Hydrogen generated by electrolysis of water can also be utilized.

As the carbon dioxide used in the embodiment of the present invention, a carbon dioxide gas cylinder, liquid carbon dioxide, supercritical carbon dioxide, dry ice, and the like can be used.

Hydrogen gas and carbon dioxide gas may be introduced into a reaction system individually or as a mixed gas.

As a ratio of hydrogen and carbon dioxide to be used, the same amount or an excess of hydrogen based on a molar basis is preferable.

When a hydrogen cylinder is used as the hydrogen used in the method for producing a formic acid according to the embodiment of the present invention, a pressure is preferably 0.1 MPa or more, more preferably 0.2 MPa or more, and still more preferably 0.5 MPa or more, from the viewpoint of achieving sufficient reactivity. The pressure is preferably 50 MPa or less, more preferably 20 MPa or less, and still more preferably 10 MPa or less as the size of the equipment tends to be large.

A pressure of carbon dioxide used in the method for producing a formic acid according to the embodiment of the present invention is preferably 0.1 MPa or more, more preferably 0.2 MPa or more, and still more preferably 0.5 MPa or more, from the viewpoint of achieving sufficient reactivity. The pressure is preferably 50 MPa or less, more preferably 20 MPa or less, and still more preferably 10 MPa or less as the size of the equipment tends to be large.

(Reaction Conditions)

Reaction conditions in the method for producing a formic acid according to the embodiment of the present invention are not limited, and the reaction conditions can be appropriately changed in the reaction process. A form of a reaction vessel used for the reaction is not limited.

A reaction temperature is not limited, but is preferably 30°C or higher, more preferably 40°C or higher, and still more preferably 50°C or higher, in order to allow the reaction to proceed efficiently. From the viewpoint of energy efficiency, the temperature is preferably 200°C or lower, more preferably 150°C or lower, and still more preferably 100°C or lower.

A reaction time is not limited, but is, for example, preferably 0.5 hours or more, more preferably 1 hour or more, and still more preferably 2 hours or more from the viewpoint of obtaining the sufficient amount of formic acid to be generated. From the viewpoint of cost, the reaction time is preferably 24 hours or less, more preferably 12 hours or less, and still more preferably 6 hours or less.

A method of introducing carbon dioxide, hydrogen, a catalyst, a solvent, and the like used for the reaction into the reaction vessel is not limited, but all the raw materials and the like may be introduced collectively, some or all the raw materials may be introduced stepwise, or some or all the raw materials may be introduced continuously. An introduction method in which these methods are combined may be used.

[Second Step]

The second step according to the embodiment of the present invention is a step of separating the amine to which the formic acid is adsorbed and the catalyst-containing solution. That is, the second step is a step of performing solid-liquid separation. In the first step, an amine immobilized on a solid is used as the amine to which the formic acid is adsorbed, and thus the catalyst-containing solution and the amine to which the formic acid is adsorbed can be separated by simple solid-liquid separation as a separation operation.

The catalyst-containing solution separated in the second step contains the catalyst and the solvent.

The separation step is not limited, and a known solid-liquid separation method can be used. Examples of the method include filtration using a filter, centrifugation, and decantation.

The catalyst-containing solution (catalyst solution) separated in the second step can be reused in the first step as a reaction solution in the first step. The catalyst usually has low durability, and is difficult to maintain the catalytic activity at the time of post-treatment such as purification after the generation of the formic acid. However, the generated formic acid and the catalyst can be easily separated, since the amine used in the first step is an amine immobilized on a solid. As a result, a decrease in catalytic activity can be prevented and the catalyst can be reused.

When the catalyst solution separated in the second step is reused, it is preferable to replenish the raw materials lost in the first step.

In the solution used in the first step, a ratio of the catalyst solution separated in the second step to be used is not limited, and the catalyst solution separated in the second step may be used as a part of the catalyst solution in the first step, or the catalyst solutions separated in the second step may be used as the entirety of the catalyst solution in the first step. However, the ratio of the catalyst solution separated in the second step to be used is preferably 50% by mass or more, and more preferably 70% by mass or more, and is preferably as high as possible from the viewpoint of the catalyst cost.

[Third Step]

The third step according to the embodiment of the present invention is a step of heating the amine to which the formic acid is adsorbed to recover the formic acid.

By heating the amine to which the formic acid is absorbed, the salt of formic acid and amine is decomposed. As a result, the formic acid can be recovered as a mixture containing the formic acid as a main component.

The amine to which the formic acid is absorbed contains the solvent even after solid-liquid separation, since the amine gets swollen with the solvent at the time of absorbing formic acid.

A temperature and a pressure of the heat treatment are not limited, and by adjusting the temperature and pressure, a mixture containing the solvent in the adsorption step of formic acid as a main component and a mixture containing the formic acid as a main component can be fractionated, and a polymer on which an amine is immobilized remains in a heating furnace.

In order to fractionate the mixture containing the solvent in the absorption step of formic acid as a main component, the temperature of the heat treatment is preferably 100°C or more, more preferably 120°C or more, and still more preferably 140°C or more, from the viewpoint of efficiently recovering the formic acid. From the viewpoint of heat resistance of the solid amine, the temperature is preferably 220°C or lower, more preferably 200°C or lower, and still more preferably 180°C or lower.

The pressure in the heat treatment is preferably 0.1 mmHg or more, more preferably 0.5 mmHg or more, and still more preferably 1 mmHg or more, from the viewpoint of efficiently recovering the formic acid. The pressure is preferably 300 mmHg or less, more preferably 250 mmHg or less, and still more preferably 200 mmHg or less.

The mixture containing the fractionated amine as a main component may be reused as the amine in the first step by being purified by a known method.

In order to fractionate the mixture containing the formic acid as a main component, the temperature of the heat treatment is preferably 60°C or more, more preferably 70°C or more, and still more preferably 80°C or more, from the viewpoint of efficiently recovering the formic acid. From the viewpoint of preventing the formic acid from decomposing, the temperature is preferably 200°C or lower, more preferably 150°C or lower, and still more preferably 120°C or lower.

The pressure in the heat treatment is preferably 100 mmHg or less, and more preferably 10 mmHg or less, from the viewpoint of the recovery efficiency of formic acid.

The polymer on which the amine remaining in the heating furnace is immobilized may be reused in the first step.

A time of the heat treatment is, for example, preferably 0.5 hours or more and 24 hours or less, more preferably 1 hour or more and 12 hours or less, and still more preferably 1 hour or more and 6 hours or less when the temperature is in the above range.

An atmosphere in the heat treatment is not limited, and the heat treatment can be performed in air.

The method of the present embodiment allows the formic acid generated in the reaction solution to be separated from the catalyst by a simple operation, and allows an expensive catalyst to be reused. The method of the present embodiment also allows the formic acid solution to be concentrated much more efficiently than a method of concentrating formic acid by an operation such as extraction or distillation from the reaction solution.

Hereinafter, the present invention will be described in detail with reference to Examples and Comparative Examples. However, the present invention is not limited to these Examples.

[Example]

[Synthesis of Catalyst]

(Synthesis Example 1) Synthesis of Ru Catalyst

A Ru catalyst was synthesized by the following operation with reference to a method for synthesizing a compound 4b described in Angew. Chem. Int. Ed. 2010, 49, 1468-1471.

Under an argon environment, 95.3 mg of [RuHCl(PPh$_3$)$_3$(CO)] (Wako Pure Chemical Industries, Ltd., Code 030-21721), 40.0 mg of 2,6-bis(di-tert-butylphosphinomethyl)pyridine (ABCR Code AB249204), and 5.0 mL of THF (tetrahydrofuran, stabilizer free, deoxygenated, Wako Pure Chemical Industries, Ltd., Code 206-18531) were added to 50 mL of Schlenk.

The Schlenk containing the mixture was placed in an oil bath at 65°C, the mixture was allowed to react for 3 hours, and then cooled to a room temperature (25°C).

The generated precipitate was filtered, and the solvent in the filtrate was distilled off using an evaporator.

The resulting yellow liquid was dissolved in a very small amount of THF and recrystallized with hexane to obtain a yellow crystal (Ru catalyst).

(Synthesis Example 2) Synthesis of Ir Catalyst

[Cp*Ir(H$_2$O)$_3$](SO$_4$) was produced by a method in Reference Example 1 described in JP-A-2008-184398. A mixture of Ag$_2$SO$_4$ (1.05 g) and [Cp*IrCl$_2$]$_2$ (1.34 g) in water (12 ml) was stirred at a room temperature for 12 hours and then filtered to remove AgCl. The solvent was distilled off under reduced pressure using an evaporator to obtain a target product ([Cp*Ir(H$_2$O)$_3$](SO$_4$)) as a yellow solid.

2.39 g of [Cp*Ir(H$_2$O)$_3$](SO$_4$) obtained above, 0.76 g of 4-hydroxy-pyridine-2-carboxylic acid methylamide (J & W Pharm Code 69R0942), and 50 mL of water were mixed and stirred at a room temperature (25°C) for 12 hours. Water was distilled off using an evaporator to obtain a yellow powder. The powder was dissolved in a very small amount of methanol and reprecipitated with ethyl acetate to obtain a target product (Ir catalyst).

[Production of Formic Acid]

[Example 1]

2.5 mg of the Ru catalyst obtained in Synthesis Example 1 was dissolved in 20 mL of dimethylformamide (DMF, Wako Pure Chemical Industries, Ltd., Code 045-32365). The catalyst solution was taken in an amount of 1.0 mL, and 1.0 mL of the catalyst solution was diluted 100 times with 99.0 mL of dimethylformamide. 20 mL of the diluted catalyst solution and 1.09 g of styrene polymer-supported 4-(dimethylamino)pyridine (Aldrich Code 359882) (PS-DMAP) were placed in a pressure-resistant container. A mixed gas having a molar ratio of CO$_2$/H$_2$ of 1/1 was added, and the pressure was set to 0.4 MPa. The mixture was allowed to react with being heated and stirred at 80°C for 1 hour.

After the reaction, the mixture was separated into a liquid phase and a solid by filtration under reduced pressure. The liquid phase was diluted with 10 times the amount of water, and HPLC analysis was performed to quantify the amount of the formic acid present in the liquid phase out of the amount of the generated formic acid, that is, the amount (mg) of the formic acid in the liquid, and further, the formic acid concentration (%) (formic acid concentration in the liquid) in the catalyst solution after the reaction was calculated. The solid was immersed in a 100 times the amount of 0.6% phosphoric acid aqueous solution, and HPLC analysis on the amount of eluted formic acid was performed to quantify the amount (mg) of formic acid to be adsorbed to the polymer, and a formic acid adsorption rate (%) to the polymer and the formic acid concentration (%) in the polymer were calculated.

HPLC was performed under the following conditions. As a standard substance for quantification, a formic acid aqueous solution adjusted to have a formic acid (manufactured by Wako Pure Chemical Industries, Ltd.) concentration of 0.001% by mass to 1% by mass was used.

Apparatus: LC-MS2010EV (manufactured by Shimadzu Corporation)

Column: YMC-Triart C18 (3.0 mm$\varphi$ $\times$ 15 cm, average particle diameter: 5 $\mu$m, average pore diameter: 12 nm)
Column temperature: 37°C
Mobile phase: solution A 0.1% $H_3PO_4$: acetonitrile = 95:5 (volume ratio),
Solution B: acetonitrile
Gradient conditions: 0 to 5 minutes, solution B 0% (hold) $\rightarrow$ 5 minutes to 5.01 minutes, solution B 0% to 95% (gradient) $\rightarrow$ 5.01 minutes to 10 minutes, solution B 95% (hold) $\rightarrow$ 10 minutes to 10.01 minutes, solution B 95% to 0% (gradient) $\rightarrow$ 10.01 minutes to 20 minutes, solution B 0% (hold)
Flow rate: 0.425 mL/min
Detection: UV 210 nm

The solid separated from the liquid phase as described above is put into a flask. A pressure was set to 100 mmHg, a temperature was set to 120°C, and a mixture containing DMF as a main component was first fractionated from the solid. Thereafter, the pressure was set to 10 mmHg and the temperature was set to 170°C, and a mixture containing a formic acid as a main component was fractionated.

The formic acid concentration (formic acid concentration (%) in the third step) in the mixture containing a formic acid as a main component was measured by HPLC, and the formic acid recovery rate (%) in the third step was calculated.

The resulting mixture was distilled under reduced pressure to obtain the formic acid at a high concentration.

First, by setting the temperature to 100°C under normal pressure, a small amount of water was distilled off. Thereafter, a formic acid solution having a high concentration was obtained by setting the pressure to 10 mmHg and the temperature to 170°C.

The formic acid concentration in the formic acid solution was measured by HPLC, and the total formic acid recovery rate (%) was calculated. The total formic acid recovery rate (%) was calculated by the following formula.

$$\text{Total formic acid recovery rate (\%)} = \text{Formic acid adsorption rate on polymer in hydrogenation} \times \text{Formic acid recovery rate in the third step)}$$

[Example 2]

A formic acid was produced and the total formic acid recovery rate was calculated in the same manner as in Example 1 except that the amount of polymer-supported 4-(dimethylamino)pyridine to be used was changed to 2.00 g.

[Example 3]

A formic acid was produced and the total formic acid recovery rate was calculated in the same manner as in Example 1 except that the amount of polymer-supported 4-(dimethylamino)pyridine to be used was changed to 4.00 g.

[Example 4]

A formic acid was produced and the total formic acid recovery rate was calculated in the same manner as in Example 1, except that 1.09 g of polymer-supported 4-(dimethylamino)pyridine was changed to 1.0 g of styrene polymer-supported 1,8-diazabicyclo[5.4.0]undec-7-ene, polymer-bound (Aldrich Code 595128) (PS-DBU).

[Example 5]

A formic acid was produced and the total formic acid recovery rate was calculated in the same manner as in Example 1 except that 1.09 g of the polymer-supported 4-(dimethylamino)pyridine was changed to 1.0 g of silica-supported alkanol-8-diazabicyclo[5.4.0]-7-undecene (DBUOH-Sillca).

[Example 6]

3.0 mg of the Ir catalyst obtained in Synthesis Example 2 was dissolved in 20 mL of water. The solution was placed in a pressure-resistant container with 1.09 g of styrene polymer-supported 4-(dimethylamino)pyridine (Aldrich Code 359882) (PS-DMAP). A mixed gas of $CO_2/H_2$ of 1/1 was introduced, and a pressure was set to 0.4 MPa. The mixture was heated and stirred at 50°C for 3 hours. After the reaction, the mixture was separated into a liquid phase and a solid by filtration under reduced pressure. The liquid phase was diluted with 10 times the amount of water, and HPLC analysis was performed to quantify the amount of formic acid. The solid was immersed in a 100 times the amount of 2% hydrochloric acid aqueous solution, and the amount of eluted formic acid was analyzed by HPLC to quantify the amount of adsorbed formic acid.

The solid separated from the liquid phase as described above was put into a flask. A pressure was set to 200 mmHg, a temperature was set to 100°C, and a mixture containing water as a main component was first fractionated from the solid. Thereafter, the total formic acid recovery rate was calculated in the same manner as in Example 1 except that the pressure was set to 10 mmHg, the temperature was set to 170°C and the mixture containing formic acid as a main component was fractionated.

[Example 7]

A formic acid was produced and the total formic acid recovery rate was calculated in the same manner as in Example 6 except that 1.09 gg of styrenepolymer-supported 4-(dimethylamino)pyridine was changed to 2.0 g of styrene polymer-supported 1,8-diazabicyclo[5.4.0]undec-7-ene, polymer-bound (Aldrich Code 595128) (PS-DBU).

[Example 8]

After performing Example 2, 14 mL of the catalyst solution recovered by filtering the solid amine was placed in a pressure-resistant container, 2.00 g of styrene polymer-supported 4-(dimethylamino)pyridine (PS-DMAP) was added, a mixed gas of $CO_2/H_2$ of 1/1 was added, and a pressure was set to 0.4 MPa. The mixture was allowed to be heated and stirred at 80 °C for 1 hour to react and a formic acid was produced, and the total formic acid recovery rate was calculated as in Example 1. This revealed that the catalyst solution can be reused.

[Comparative Example 1]

2.5 mg of the Ru catalyst obtained in Synthesis Example 1 and 1.69 g of 1,8-diazicyclo[5.4.0]undec-7-ene (DBU TCI Code D1270) were dissolved in 20 mL of DMF. As a solution for dilution, a mixture of 8.45 g of 1,8-diazicyclo[5.4.0]undec-7-ene (DBU TCI Code D1270) and 100 mL of DMF was prepared. The catalyst solution was diluted 100 times with the solution for dilution. The diluted catalyst solution was placed in a 20 mL pressure-resistant container. A mixed gas of $CO_2/H_2$ of 1/1 was introduced, and a pressure was set to 0.4 MPa. The mixture was heated and stirred at a reaction temperature of 80°C for 1 hour. The catalyst solution after the reaction was diluted 10 times with a 20% hydrochloric acid aqueous solution, and HPLC analysis was performed to quantify the amount of formic acid in the catalyst solution after the reaction.

The resulting catalyst solution was not concentrated because the formic acid and the solvent caused azeotropy, and the amount of the recovered formic acid and the total formic acid recovery rate were not obtained.

[Comparative Example 2]

A formic acid was produced in the same manner as in Comparative Example 1 except that a reaction temperature was changed to 50°C.

The resulting catalyst solution was not concentrated because the formic acid and the solvent caused azeotropy, and the amount of the recovered formic acid and the total formic acid recovery rate were not obtained.

[Comparative Example 3]

3.0 mg of the Ir catalyst obtained in Synthesis Example 2 was dissolved in 20 mL of a 2 mol/L potassium hydrogencarbonate aqueous solution. The diluted catalyst solution was placed in a 20 mL pressure-resistant container. A mixed gas of $CO_2/H_2$ of 1/1 was introduced, and a pressure was set to 0.4 MPa. The mixture was heated and stirred at 50°C for 3 hours. The catalyst solution after the reaction was diluted 10 times with a 20% hydrochloric acid aqueous solution, and HPLC analysis was performed to quantify the amount of formic acid.

The resulting catalyst solution was not concentrated because the formic acid and the solvent caused azeotropy, and the amount of the recovered formic acid and the total formic acid recovery rate were not obtained.

The above examples and comparative examples are shown in Table 1.

TOF (TurnOver Frequency) in the table indicates a molar amount of formic acid or formate generated per hour with respect to a molar amount of the used catalyst. [0053]

[Table 1]

| | | Example 1 | Example 2 | Example 3 | Example 4 | Example 5 | Example 6 | Example 7 | Example 8 | Comparative Example 1 | Comparative Example 2 | Comparative Example 3 |
|---|---|---|---|---|---|---|---|---|---|---|---|---|
| $CO_2$ hydrogenation | Catalyst | Ru | Ru | Ru | Ru | Ru | Ir | Ir | Ru | Ru | Ru | Ir |
| | Catalyst amount umol | 0.05 | 0.05 | 0.05 | 0.05 | 0.05 | 5 | 5 | 0.035 | 0.05 | 0.05 | 5 |
| | Solvent | DMF | DMF | DMF | DMF | DMF | Water | Water | DMF | DMF | DMF | Water |
| | Base type | PS-DMAP | PS-DMAP | PS-DMAP | PS-DBU | DBUOH-Silica | PS-DMAP | PS-DBU | PS-DMAP | DBU | DBU | KHCO$_3$ |
| | Solvent amount mL | 20 | 20 | 20 | 20 | 20 | 20 | 20 | 14 | 20 | 20 | 20 |
| | Base-supported amount mmol/g (in case of polymer or silica) | 5.5 | 5.5 | 5.5 | 1.0 | 0.5 | 5.5 | 1.0 | 5.5 | - | - | - |
| | Polymer or silica amount g | 1.09 | 2.00 | 4.00 | 1.00 | 1.00 | 1.09 | 2.00 | 2.00 | - | - | - |
| | Total base amount mmol | 6.0 | 11.0 | 22.0 | 1.0 | 0.5 | 6.0 | 2.0 | 11.0 | 11.0 | 11.0 | 40.0 |
| | Reaction conditions | 0.4 MPa 80°C | 0.4 MPa 80°C | 0.4 MPa 80°C | 0.4 MPa 80°C | 0.4 MPa 80°C | 0.4 MPa 50°C | 0.4 MPa 50°C | 0.4 MPa 80°C | 0.4 MPa 80°C | 0.4 MPa 50°C | 0.4 MPa 50°C |
| | Reaction time hr | 1 | 1 | 1 | 1 | 1 | 3 | 3 | 1 | 1 | 1 | 3 |
| | TOF h-1 | 11,000 | 14,000 | 28,000 | 8,000 | 1,900 | 16 | 12 | 14,000 | 176,000 | 199,000 | 277 |
| | Formic acid generation amount mg | 25.3 | 32.2 | 64.4 | 18.4 | 4.4 | 11.0 | 8.3 | 22.5 | 404.8 | 457.7 | 191.1 |
| | Formic acid amount mg in liquid | 17.3 | 11.3 | 22.5 | 17.0 | 0.0 | 3.5 | 1.0 | 7.9 | 404.8 | 457,7 | 191.1 |

| | | Example 1 | Example 2 | Example 3 | Example 4 | Example 5 | Example 6 | Example 7 | Example 8 | Comparative Example 1 | Comparative Example 2 | Comparative Example 3 |
|---|---|---|---|---|---|---|---|---|---|---|---|---|
| | Formic acid concentration % in liquid | 0.09 | 0.06 | 0.11 | 0.09 | 0.00 | 0.02 | 0.00 | 0.06 | 2.15 | 2.43 | 0.96 |
| | Formic acid adsorption rate % on polymer or silica | 31.8 | 65.0 | 65.0 | 7.5 | 100.0 | 68.8 | 88.0 | 65.0 | - | - | - |
| | Formic acid adsorption amount mg on polymer or silica | 8.1 | 20.9 | 41.9 | 1.4 | 4.4 | 7.6 | 7.3 | 14.7 | - | - | - |
| | Formic acid concentration % in polymer or silica | 0.7 | 1.0 | 1.0 | 0.1 | 0.4 | 0.7 | 0.4 | 0.7 | - | - | - |
| Formic acid recovery (the third step) | Formic acid recovery rate % | 14 | 17 | 20 | 8 | 9 | 16 | 10 | 17 | - | - | - |
| | Formic acid concentration % | 57 | 60 | 62 | 50 | 52 | 65 | 55 | 60 | - | - | - |
| Total | Formic acid recovery amount mg | 1.13 | 3.56 | 8.37 | 0.11 | 0.39 | 1.21 | 0.73 | 2.49 | - | - | - |
| | Total formic acid recovery rate % | 4.5 | 11.1 | 13.0 | 0.6 | 9.0 | 11.0 | 8.8 | 11.1 | - | - | - |
| (Total formic acid recovery rate = Formic acid adsorption rate on polymer or silica in hydrogenation x Formic acid recovery rate in the third step) | | | | | | | | | | | | |

EP 3 936 498 A1

In Examples 1 to 7, using the amine immobilized on the solid in the first step allowed the amine to which the formic acid was absorbed and the catalyst-containing solution to be separated by a simple method.

As can be seen from Table 1, in Examples 1 to 7, separating the generated formic acid from the catalyst-containing solution allowed the formic acid to be recovered as a formic acid mixed solution in high concentration and the formic acid can be further concentrated by distillation, so that the total formic acid recovery rate is high. In Example 8, as a result of using the catalyst solution recovered by filtering the solid amine in Example 2, it was revealed that the same total formic acid recovery rate as in Example 2 was obtained and the catalyst solution could be reused.

On the other hand, in Comparative Examples 1 to 3, the generated formic acid was extracted with a liquid amine and thus the formic acid and the solvent caused azeotropy. As a result, the resulting formic acid solution could not be concentrated by distillation.

Although the present invention are described in detail with reference to the specific embodiment, it will be apparent to those skilled in the art that various changes and modifications can be made without departing from the spirit and scope of the present invention.

The present application is based on a Japanese Patent Application (Patent Application No. 2019-42918) filed on March 8, 2019, and a Japanese Patent Application (Patent Application No. 2019-227277) filed on December 17, 2019, contents of which are incorporated herein by reference.

INDUSTRIAL APPLICABILITY

The present invention provides a method for producing a formic acid that can concentrate a formic acid solution with high efficiency by a simple method and recover a formic acid at a high yield.

**Claims**

1. A method for producing a formic acid, comprising:

   a first step of allowing carbon dioxide and hydrogen to react with each other in a solution containing a solvent and a catalyst dissolved in the solvent and in the presence of an amine insoluble in the solvent, and allowing a generated formic acid to adsorb to the amine, wherein
   the catalyst contains at least one metal element selected from the group consisting of metal elements belonging to Groups 8, 9, and 10 of a periodic table, and
   the amine is an amine immobilized on a solid.

2. The method for producing a formic acid according to claim 1, further comprising: a second step of separating the amine to which the formic acid is adsorbed and the catalyst-containing solution.

3. The method for producing a formic acid according to claim 2, wherein the catalyst-containing solution separated in the second step is reused in the first step.

4. The method for producing a formic acid according to claim 2 or 3, further comprising:
   a third step of heating the amine to which the formic acid is adsorbed to recover the formic acid.

5. The method for producing a formic acid according to any one of claims 1 to 4, wherein the metal element is Ru, Ir, Fe, or Co.

6. The method for producing a formic acid according to any one of claims 1 to 5, wherein the metal element is Ir or Ru.

7. The method for producing a formic acid according to any one of claims 1 to 6, wherein the amine immobilized on the solid is an amine immobilized on a polymer.

8. The method for producing a formic acid according to claim 7, wherein the polymer on which the amine is immobilized is polystyrene.

## INTERNATIONAL SEARCH REPORT

| International application No. |
|---|
| PCT/JP2020/008680 |

**A. CLASSIFICATION OF SUBJECT MATTER**
Int.Cl. C07C51/00(2006.01)i, C07C53/02(2006.01)i, C07B61/00(2006.01)i,
B01J31/22(2006.01)i, B01J31/24(2006.01)i
FI: C07C51/00, C07C53/02, C07B61/00300, B01J31/24Z, B01J31/22Z
According to International Patent Classification (IPC) or to both national classification and IPC

**B. FIELDS SEARCHED**

Minimum documentation searched (classification system followed by classification symbols)
Int.Cl. C07C51/00, C07C53/02, C07B61/00, B01J31/22, B01J31/24

| Documentation searched other than minimum documentation to the extent that such documents are included in the fields searched | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2015-502922 A (BASF SE) 29.01.2015 (2015-01-29), entire text, all drawings | 1-8 |
| A | JP 2010-521533 A (BASF SE) 24.06.2010 (2010-06-24), entire text | 1-8 |
| A | JP 60-209260 A (BP CHEMICALS LIMITED) 21.10.1985 (1985-10-21), entire text | 1-8 |

☐ Further documents are listed in the continuation of Box C.      ☒ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 19.05.2020 | 02.06.2020 |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

| | | | |
|---|---|---|---|
| **INTERNATIONAL SEARCH REPORT** | | International application No. | |
| Information on patent family members | | PCT/JP2020/008680 | |

| | | |
|---|---|---|
| JP 2015-502922 A | 29.01.2015 | WO 2013/068389 A1 |
| | | entire text, all drawings |
| | | EP 2776448 A1 |
| | | CN 103917551 A |
| | | KR 10-2014-0090183 A |
| | | |
| JP 2010-521533 A | 24.06.2010 | US 2010/0063320 A1 |
| | | entire text |
| | | WO 2008/116799 A1 |
| | | EP 2139837 A1 |
| | | KR 10-2009-0123972 A |
| | | CN 101663259 A |
| | | |
| JP 60-209260 A | 21.10.1985 | US 4879070 A |
| | | entire text |
| | | EP 151510 A1 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8791297 B
- JP 2008184398 A
- JP 2019042918 A
- JP 2019227277 A

**Non-patent literature cited in the description**

- *RSC Adv.,* 2014, vol. 4, 49995-50002
- *Angew. Chem. Int. Ed.,* 2010, vol. 49, 1468-1471